# EUROPEAN PATENT APPLICATION

(11) **EP 4 848 116 A1**
(43) Date of publication of application: **02.12.2026**
(21) Application number: 25767402.8
(22) Date of filing: 05.03.2025
(51) Int. Cl.: G06F 18/22, G16C 20/50, G16C 20/70

(54) **MOLECULAR SIMILARITY CALCULATION METHOD AND APPARATUS BASED ON PHOTONIC QUANTUM COMPUTER**

(30) Priority: 06.03.2024 CN 202410251527
(71) Applicant: Beijing QBoson Quantum Technology Co., Ltd, Beijing 100015 (CN)
(72) Inventor: WEN, Kai, Beijing 100015 (CN); MA, Yin, Beijing 100015 (CN)
(74) Representative: Grünecker Patent- und Rechtsanwälte PartG mbB
(86) International application number: PCT/CN2025/080813
(87) International publication number: WO 2025/185664

(57) **Abstract**

The present invention relates to the technical field of drug design, and in particular to a molecular similarity calculation method based on a photonic quantum computer, comprising: on the basis of molecular structures, respectively converting two molecules requiring molecular similarity calculation into molecular graphs, wherein vertices of each molecular graph represent atoms in the corresponding molecule, and edges of the molecular graph represent chemical bond connections between the atoms in the molecule; using the two molecular graphs to construct a conflict graph, wherein vertices of the conflict graph are derived from pairwise vertex matching mappings between the two molecular graphs, and edges of the conflict graph are derived from discrimination results between the vertices in the conflict graph; solving a maximum weighted independent subset of the conflict graph by means of a photonic quantum computer and obtaining information of each vertex of the conflict graph in the maximum weighted independent subset; and calculating the similarity between the two molecules on the basis of the information of each vertex of the conflict graph in the maximum weighted independent subset. The method overcomes the defects in conventional solving approaches of low solving speed and high false positive rate, effectively improving the calculation speed and accuracy.

## Description

### FIELD OF TECHNOLOGY

The present disclosure relates to the technical field of drug design, and relates to a molecular similarity calculation method and apparatus, and particularly relates to a molecular similarity calculation method and apparatus based on photonic quantum computer.

### BACKGROUND

In the field of drug design, drug development is costly, time-consuming, and has a high failure rate. Therefore, researchers have been searching for more cost-effective drug development methods with a higher probability of success. Traditional drug discovery can be divided into receptor-based and ligand-based drug screening. Among them, ligand-based drug screening mainly screens molecules similar to known active molecule groups as lead compounds, and then further screens and verifies them as drug candidate molecules.

Existing methods compare the similarity of different molecules through molecular fingerprints. Molecular fingerprints are created by encoding molecular structure fragments into characteristic binary vectors, where each bit corresponds to the presence of a specific fragment. If two molecular fingerprints have a 1 at the same position, then both molecules have the same fragment, and the more fragments they share, the more similar they are. This method compares the similarity of molecules at the 2D level, but does not use all the valid information contained in the molecule, such as the 3D conformation of the molecule. The 3D conformation of a molecule has an important influence when binding to proteins in a real reaction system, and the molecular similarity obtained based on the molecular fingerprint method will lose this information. Therefore, the similarity calculation through molecular fingerprints leads to a high false positive rate, that is, the highly similar compounds screened out through calculation do not have activity in real experimental systems, and it is time-consuming to screen in compound libraries with a large number of molecules.

Therefore, in view of the defects existing in the prior art, there is a need to develop a novel molecular similarity calculation method and apparatus.

### SUMMARY

In order to overcome the defects of the prior art, the present disclosure provides a molecular similarity calculation method and apparatus based on photonic quantum computer, which solves the disadvantages of slow solving speed and high false positive rate of traditional solving methods, and effectively improves the calculation speed and accuracy.

To achieve the above objective, the present disclosure provides the following technical solutions.

A molecular similarity calculation method based on photonic quantum computer, comprising:
converting two molecules requiring molecular similarity calculation into molecular graphs respectively based on molecular structures, wherein vertices of the molecular graph represent respective atoms in the molecule, and edges of the molecular graph represent chemical bond connections between respective atoms in the molecule;
constructing a conflict graph utilizing the two molecular graphs, wherein vertices of the conflict graph are derived from pairwise matching mappings between vertices in the two molecular graphs, and edges of the conflict graph are derived from discrimination results between respective vertices in the conflict graph;
solving a maximum weighted independent subset of the conflict graph by the photonic quantum computer and obtaining information of respective vertices of the conflict graph in the maximum weighted independent subset;
calculating the similarity of the two molecules through the information of respective vertices of the conflict graph in the maximum weighted independent subset.

Preferably, the vertices of the molecular graph contain characteristic information of atoms and the edges of the molecular graph contain chemical bond connection information between atoms, wherein the characteristic information of the atoms includes atom name, number of explicit hydrogens, number of implicit hydrogens, number of adjacent atoms, explicit valence, formal charge, and whether it is an atom in a ring, and the chemical bond connection information between atoms includes bond type, bond aromaticity, whether it is a bond in a ring, and bond stereo information.

Preferably, when constructing the conflict graph utilizing the two molecular graphs, matched atom pairs are obtained by comparing the similarity between respective atoms of the original molecules corresponding to the two molecular graphs, and the matched atom pairs are used as the vertices of the conflict graph; and whether mappings of two vertices in the conflict graph conflict is obtained by comparing whether a difference between distances of two atoms in the original molecules corresponding to the vertices in the two molecular graphs contained in the two vertices of the conflict graph is greater than a threshold, thereby determining whether to add an edge between the two vertices.

Preferably, obtaining matched atom pairs by comparing the similarity between respective atoms of the original molecules corresponding to the two molecular graphs, and using the matched atom pairs as the vertices of the conflict graph specifically comprises:
calculating the similarity between respective atoms of the original molecules corresponding to the two molecular graphs, wherein the similarity between two atoms is calculated as follows: $similarity between two atoms = Average \left({\sum }_{n = 1}^{N} \frac{min \left({Gf}_{n},G′{f}_{n}\right)}{max \left({Gf}_{n},G′{f}_{n}\right)}\right)$ wherein, *N* represents the total number of the atom's characteristic information, *n* represents the ordinal number of the atom's characteristic information, *Gfₙ* and *G'fₙ* respectively represent a value of an n-th characteristic information of the atom in the original molecule corresponding to a first molecular graph and a value of an *n*-th characteristic information of the atom in the original molecule corresponding to a second molecular graph, min(*Gfₙ*, *G'fₙ*) represents a smaller value between the value of the *n*-th characteristic information of the atom in the original molecule corresponding to the first molecular graph and the value of the n-th characteristic information of the atom in the original molecule corresponding to the second molecular graph, and *max*(*Gfₙ, G'fₙ*) represents a larger value therebetween;
comparing the similarity between the two atoms with a threshold *S*, and when it is greater than the threshold *S*, determining that a mapping exists between the atom in the original molecule corresponding to the first molecular graph and the atom in the original molecule corresponding to the second molecular graph, whereby these two atoms are a matched atom pair, and taking the matched atom pair as a vertex of the conflict graph;
performing pairwise atom similarity calculations on all atoms in the original molecule corresponding to the first molecular graph and all atoms in the original molecule corresponding to the second molecular graph to find all matched atom pairs, and obtaining a vertex set with vertex weights $\left\{\left(vi,v′j\right)\left|{\sum }_{n = 1}^{N} {Gf}_{n} + G ′ {f}_{n}\right.\right\}$ of the conflict graph, wherein *vᵢ* represents the *i*-th atom in the original molecule corresponding to the first molecular graph, *v'ⱼ*. represents the *j*-th atom in the original molecule corresponding to the second molecular graph, and ${\sum }_{n = 1}^{N} {Gf}_{n} + G ′ {f}_{n}$ represents the vertex weight of the vertex in the conflict graph.

Preferably, the threshold *S* is 0.75.

Preferably, obtaining whether mappings of two vertices in the conflict graph conflict by comparing whether the difference between the distances of two atoms in the original molecules corresponding to the vertices in the two molecular graphs contained in the two vertices of the conflict graph is greater than a threshold, thereby determining whether to add an edge between the two vertices, specifically comprises:
comparing whether the difference between the distances of the two atoms in the original molecules corresponding to the vertices in the two molecular graphs contained in the two vertices of the conflict graph is greater than a threshold *dₜ,* and if it is greater than the threshold *dₜ,* adding an edge between the two vertices, wherein a specific mathematical expression for the determination is as follows: ${E}_{c} = \left\{\left.\left({v}_{i},v{′}_{j}\right), \left({v}_{k},v{′}_{l}\right)\right|\left|d\left({v}_{i}, {v}_{k}\right)-d\left(v{′}_{j},v{′}_{l}\right)\right|>{d}_{t}\right\}$ Wherein, *E_{c}* represents an edge set between the vertices of the conflict graph, (*vᵢ*, *v'ⱼ*) and (*vₖ, v'ₗ*) represent two vertices in the conflict graph, and *d*(*vᵢ, vₖ*) and *d*(*v'ⱼ, v'ₗ*) respectively represent a geometric distance between two atoms in the original molecule corresponding to the vertices *vᵢ* and *vₖ* in the first molecular graph and a geometric distance between two atoms in the original molecule corresponding to the vertices *v'ⱼ*. and *v'ₗ* in the second molecular graph.

Preferably, the threshold *dₜ* is 2, and an edge weight is assigned to an edge of the conflict graph, wherein a value of the edge weight is a smaller value of the vertex weights of the two connected vertices plus 1.

Preferably, when solving the maximum weighted independent subset of the conflict graph, a QUBO model as follows is constructed: $H = min \left(-{\sum }_{p ∈ Vc} {w}_{p} {x}_{p} + {\sum }_{\left(p, q\right) ∈ {E}_{c}} {w}_{pq} {x}_{p} {x}_{q}\right)$ wherein, *H* represents the Hamiltonian, which is an optimization target value; *p* and q respectively represent a *p*-th vertex and a *q*-th vertex in the conflict graph; *xₚ* and *x_{q}* are two binary variables, if the p-th vertex and the *q*-th vertex of the conflict graph are respectively included in the maximum weighted independent subset, *xₚ* and *x_{q}* are equal to 1, otherwise *xₚ* and *x_{q}* are equal to 0; *wₚ* is the vertex weight of the *p*-th vertex in the conflict graph; *w_{pq}* represents the edge weight of the edge connecting the *p*-th vertex and the *q*-th vertex in the conflict graph; and *V_{c}* and *E_{c}* respectively represent the vertex set and the edge set in the conflict graph.

Preferably, a specific calculation method for calculating the similarity of the two molecules through the information of respective vertices of the conflict graph in the maximum weighted independent subset is as follows: $S \left(G,G′\right) = δ max \left\{\frac{\left|{V}_{c}^{1}\right|}{\left|V1\right|}, \frac{\left|{V}_{c}^{2}\right|}{\left|V2\right|}\right\} + \left(1-δ\right) min \left\{\frac{\left|{V}_{c}^{1}\right|}{\left|V1\right|}, \frac{\left|{V}_{c}^{2}\right|}{\left|V2\right|}\right\} , δ ∈ \left[0, 1\right]$ wherein, *G* and *G*' represent the two molecules requiring molecular similarity calculation, *S*(*G, G'*) represents the similarity between molecule *G* and molecule *G*', |*V*1| and |*V*2| respectively represent the number of atoms of molecule *G* and molecule *G*', $\left|{V}_{c}^{1}\right|$ and $\left|{V}_{c}^{2}\right|$ respectively represent the number of atoms belonging only to molecule *G* and only to molecule *G'* in the respective vertices of the conflict graph in the maximum weighted independent subset, and *δ* is an adjustable parameter.

In addition, the present disclosure further provides a molecular similarity calculation apparatus based on photonic quantum computer, comprising:
one or more processors;
a memory, configured to store one or more programs;
wherein when the one or more programs are executed by the one or more processors, the one or more processors are caused to implement the molecular similarity calculation method based on a photonic quantum computer according to any one of claims 1-9.

Compared with the prior art, the molecular similarity calculation method and apparatus based on photonic quantum computer of the present disclosure have one or more of the following beneficial technical effects:
1.By designing a solving model suitable for a photonic quantum computer, the present disclosure solves the problem of calculating molecular similarity, overcomes the disadvantages of slow solving speed and high false positive rate of traditional solving methods, and effectively improves the calculation speed and accuracy.
2. By designing a mathematical model suitable for quantum computing and then using a photonic quantum computer to accelerate the solving of the problem, the calculation efficiency and solving result are improved, which solves the problem in the prior art that complex scheduling problems are difficult to calculate and exact solutions cannot be obtained.
3. Based on the problem that traditional solving methods can only obtain approximate solutions rather than global optimal solutions and thus cannot obtain results with high accuracy, the present disclosure models using QUBO (Quadratic Unconstrained Binary Optimization) and utilizes a photonic quantum computer to obtain the optimal solution to the problem.

### BRIEF DESCRIPTION OF THE DRAWINGS

Fig. 1 is a flowchart of the molecular similarity calculation method based on photonic quantum computer of the present disclosure.
Fig. 2 is an ROC curve solved by the photonic quantum computer for COMT class molecules.

### DESCRIPTION OF THE EMBODIMENTS

Before describing in detail any embodiments of the present disclosure, it should be understood that the present disclosure is not limited in its application to the details of construction and arrangement of components set forth in the following description or illustrated in the following drawings. The present disclosure is capable of other embodiments and of being practiced or carried out in various ways. Also, it should be understood that the phraseology and terminology used herein are for the purpose of description and should not be regarded as limiting. The use of "including" or "having" and variations thereof herein is intended to encompass the items listed hereinafter and equivalents thereof as well as additional items. Unless otherwise specified or limited, the terms "mounted," "connected," "supported," and "coupled" and variations thereof are used broadly and encompass both direct and indirect mounting, connecting, supporting, and coupling. Moreover, "connected" and "coupled" are not restricted to physical or mechanical connections or couplings.

Furthermore, in the present disclosure, the term "a" should be understood as "at least one" or "one or more," that is, in one embodiment, the number of one element may be one, while in other embodiments, the number of the element may be multiple, and the term "a" cannot be understood as a limitation on the number.

Molecular similarity is often used to screen molecules with structures similar to known active groups. The traditional molecular similarity calculation method first generates molecular fingerprints of two molecules, and obtains similarity information by comparing the molecular fingerprints of the two molecules. However, the similarity calculation method based on molecular fingerprints does not use all the valid information contained in the molecule, such as the 3D conformation of the molecule. The 3D conformation of a molecule has an important influence when binding to proteins in a real reaction system, and the molecular similarity obtained based on the molecular fingerprint method will lose this information. Therefore, the similarity calculation through molecular fingerprints leads to a high false positive rate, that is, the highly similar compounds screened out through calculation do not have activity in real experimental systems, and it is time-consuming to screen in compound libraries with a large number of molecules.

In order to overcome the above defects, in the present disclosure, utilizing the 3D information of molecules, a single molecule is first converted into a graph structure, wherein the vertices of the molecular graph represent respective atoms in the original molecule, and the edges of the molecular graph represent the chemical bond connections between respective atoms in the original molecule. In order to calculate the similarity of two molecules, a conflict graph is first constructed utilizing the two molecular graphs. The vertices of the conflict graph are derived from pairwise matching mappings between vertices in the two molecular graphs. If matching requirements are met, a vertex is generated. The edge connections in the conflict graph are derived from discrimination results between respective vertices in the conflict graph. If the difference between the geometric distances of two vertices contained in two vertices in the conflict graph in the original respective molecular graphs is greater than a threshold, an edge is added between these two vertices in the conflict graph, and this edge is assigned a weight, which is the minimum value of the weights of the two connected vertices plus 1. By constructing the conflict graph, the problem of solving molecular similarity is first converted into solving the maximum weighted independent subset of the conflict graph, and then a combinatorial optimization mathematical model suitable for quantum computing is designed to solve the problem. Finally, a photonic quantum computer is used to solve the optimal solution of the problem, obtain the maximum weighted independent subset and obtain the information of respective vertices in the set, and calculate the molecular similarity according to the vertex information.

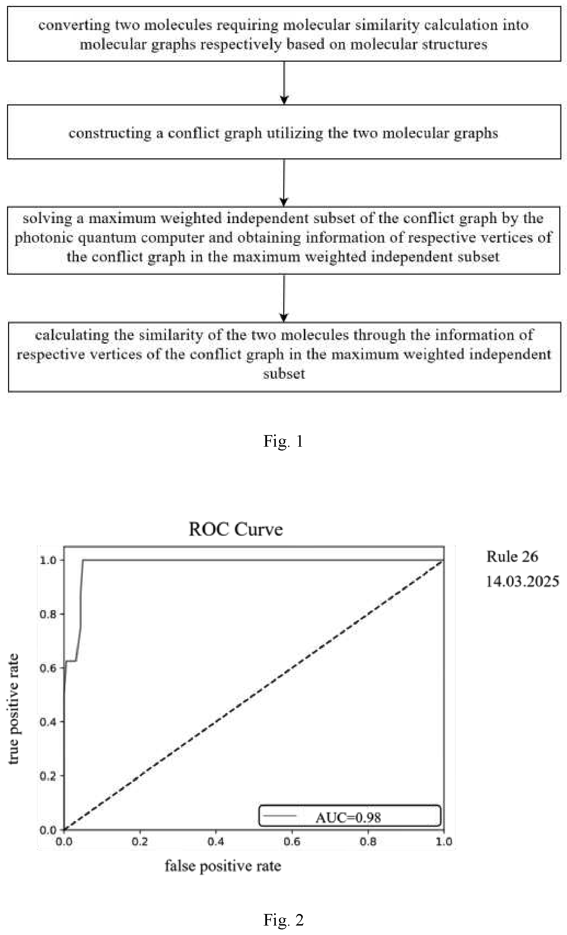

Fig. 1 shows a flowchart of the molecular similarity calculation method based on photonic quantum computer of the present disclosure. As shown in Fig. 1, the molecular similarity calculation method based on photonic quantum computer of the present disclosure includes the following steps:

### 1. Convert two molecules requiring molecular similarity calculation into molecular graphs respectively based on molecular structures.

Wherein, the vertices of the molecular graph represent respective atoms in the molecule, and the edges of the molecular graph represent chemical bond connections between respective atoms in the molecule.

For the convenience of information extraction and calculation, the present disclosure first converts a single molecule into a molecular graph representation structure based on the molecular structure. The specific conversion process is as follows: the vertices in the molecular graph are derived from respective atoms in the original molecule, and the vertices contain characteristic information of the atoms. The characteristic information is extracted by using RDKit (RDKit is an open-source toolkit for cheminformatics) based on the smiles format file of the molecule, and mainly includes: atom name, number of explicit hydrogens, number of implicit hydrogens, number of adjacent atoms, explicit valence, formal charge, and whether it is an atom in a ring. The edge connections between respective vertices in the molecular graph are derived from the chemical bond connections between respective atoms in the original molecule, and the edges in the molecular graph contain the chemical bond connection information in the original molecule, mainly including bond type (single bond, double bond, triple bond, conjugated), bond aromaticity, whether it is a bond in a ring, bond stereo information, etc.

Through the above process, the molecular graphs of the two molecules requiring molecular similarity calculation are obtained for the next step of calculation.

### 2. Construct a conflict graph utilizing the two molecular graphs.

In the first step, the molecular graphs of the two molecules requiring molecular similarity calculation are obtained, and next, a conflict graph is constructed based on the two molecular graphs.

In the present disclosure, the vertices of the conflict graph are derived from pairwise matching mappings between vertices in the two molecular graphs. That is, matched atom pairs are obtained by comparing the similarity between respective atoms of the original molecules corresponding to the two molecular graphs, and the matched atom pairs are used as the vertices of the conflict graph.

Specifically, first, the similarity between respective atoms of the original molecules corresponding to the two molecular graphs is calculated. Wherein, the similarity between two atoms is calculated as follows: $similarity between two atoms = Average \left({\sum }_{n = 1}^{N} \frac{min \left({Gf}_{n},G′{f}_{n}\right)}{max \left({Gf}_{n},G′{f}_{n}\right)}\right)$

Wherein, *N* represents the total number of the atom's characteristic information (as described above, in the present disclosure, there are seven pieces of the atom's characteristic information, namely atom name, number of explicit hydrogens, number of implicit hydrogens, number of adjacent atoms, explicit valence, formal charge, and whether it is an atom in a ring, therefore, *N=*7), *n* represents the ordinal number of the atom's characteristic information (in the present disclosure, *n* is a value from 1 to 7), *Gfₙ* and *G'fₙ* respectively represent a value of an n-th characteristic information of the atom in the original molecule corresponding to a first molecular graph and a value of an n-th characteristic information of the atom in the original molecule corresponding to a second molecular graph, min(*Gfₙ*, *G'fₙ*) represents a smaller value between the value of the n-th characteristic information of the atom in the original molecule corresponding to the first molecular graph and the value of the n-th characteristic information of the atom in the original molecule corresponding to the second molecular graph, and *max*(*Gfₙ, G'fₙ*) represents a larger value therebetween.

Second, the similarity between the two atoms is compared with a threshold *S*. When it is greater than the threshold S, it is determined that a mapping exists between the atom in the original molecule corresponding to the first molecular graph and the atom in the original molecule corresponding to the second molecular graph, whereby these two atoms are a matched atom pair, and the matched atom pair is taken as a vertex of the conflict graph. In the present disclosure, the threshold *S* is 0.75.

Next, pairwise atom similarity calculations are performed on all atoms in the original molecule corresponding to the first molecular graph and all atoms in the original molecule corresponding to the second molecular graph to find all matched atom pairs, and a vertex set with vertex weights $\left\{\left(vi,v′j\right)\left|{\sum }_{n = 1}^{N} {Gf}_{n} + G ′ {f}_{n}\right.\right\}$ of the conflict graph is obtained. Wherein, *vᵢ* represents the *i*-th atom in the original molecule corresponding to the first molecular graph, *v'ⱼ* represents the *j*-th atom in the original molecule corresponding to the second molecular graph, and ${\sum }_{n = 1}^{N} {Gf}_{n} + G ′ {f}_{n}$ represents the vertex weight of the vertex in the conflict graph. Each vertex in the vertex set represents a possible atom pairing in the original two molecules, and its vertex weight shows the contribution to the overall molecular similarity.

Next, the issue of edge connection in the conflict graph is considered. The edges of the conflict graph are derived from discrimination results between respective vertices in the conflict graph. That is, whether mappings of two vertices in the conflict graph conflict is obtained by comparing whether the difference between the distances of two atoms in the original molecules corresponding to the vertices in the two molecular graphs contained in the two vertices of the conflict graph is greater than a threshold, thereby determining whether to add an edge between the two vertices.

Specifically, whether the difference between the distances of the two atoms in the original molecules corresponding to the vertices in the two molecular graphs contained in the two vertices of the conflict graph is greater than the threshold *dₜ* is compared. If it is greater than the threshold *dₜ,* it indicates that the mappings of the two vertices in the conflict graph conflict, and an edge is added between the two vertices. The specific mathematical expression for the determination is as follows: ${E}_{c} = \left\{\left.\left({v}_{i},v{′}_{j}\right), \left({v}_{k},v{′}_{l}\right)\right|\left|d\left({v}_{i}, {v}_{k}\right)-d\left(v{′}_{j},v{′}_{l}\right)\right|>{d}_{t}\right\}$

Wherein, *E_{c}* represents an edge set between the vertices of the conflict graph, (*vᵢ, v'ⱼ*) and (*vₖ, v'ₗ*) represent two vertices in the conflict graph, and *d*(*vᵢ, vₖ*) and *d*(*v'ⱼ, v'ₗ*) respectively represent a geometric distance between two atoms in the original molecule corresponding to the vertices *vᵢ* and *vₖ* in the first molecular graph and a geometric distance between two atoms in the original molecule corresponding to the vertices *v'ⱼ* and *v'ₗ* in the second molecular graph.

In the present disclosure, while adding an edge between two vertices, an edge weight is assigned to the edge of the conflict graph, and the value of the edge weight is the smaller value of the vertex weights of the two connected vertices plus 1. Moreover, the threshold *dₜ* is a variable threshold parameter, and in the present disclosure, it is set to 2.

Through the above steps, a conflict graph with weights on both vertices and edges is obtained.

3. Solve the maximum weighted independent subset of the conflict graph by the photonic quantum computer and obtain information of respective vertices of the conflict graph in the maximum weighted independent subset.

After constructing the conflict graph, the molecular similarity solving problem can be converted into a maximum weighted independent subset problem based on the conflict graph. In the conflict graph, vertices represent possible pairwise vertex matching mappings in the original two molecules, and the edges connecting the vertices of the conflict graph represent conflict relationships existing between the vertices of the conflict graph, and both the vertices and edges of the conflict graph carry weights. Therefore, the solving objective is to find an independent vertex set that maximizes the total weight. The maximum weighted independent subset problem is a classic NP-hard problem, and it is difficult to find the optimal solution using classical computers. Establishing a QUBO model for this problem and utilizing a quantum computer to solve it is an effective solution to NP-hard problems. For solving the maximum weighted independent subset of the conflict graph in this problem, the following QUBO model can be constructed: $H = min \left(-{\sum }_{p ∈ Vc} {w}_{p} {x}_{p} + {\sum }_{\left(p, q\right) ∈ {E}_{c}} {w}_{pq} {x}_{p} {x}_{q}\right)$

Wherein, H represents the Hamiltonian, which is an optimization target value; *p* and *q* respectively represent a p-th vertex and a q-th vertex in the conflict graph; *xₚ* and *x_{q}* are two binary variables, if the p-th vertex and the q-th vertex of the conflict graph are respectively included in the maximum weighted independent subset, *xₚ* and *x_{q}* are equal to 1, otherwise *xₚ* and *x_{q}* are equal to 0; *wₚ* is the vertex weight of the p-th vertex in the conflict graph; *w_{pq}* represents the edge weight of the edge connecting the p-th vertex and the q-th vertex in the conflict graph; and *V_{c}* and *E_{c}* respectively represent the vertex set and the edge set in the conflict graph. For example, for two molecules each containing 20 atoms, the vertex set of the obtained conflict graph may have a maximum of 400, and the edge set may be 160,000.

Then, the photonic quantum computer is utilized to solve based on the above QUBO model. The QUBO model is applicable to special-purpose quantum computers such as quantum annealers and Coherent Ising Machines (CIM). The physical machine implementation solving takes the CIM based on a Degenerate Optical Parametric Oscillator (DOPO) as an example. This is a hybrid quantum computing system composed of an optical part and an electrical part. The optical part includes a laser, an amplifier, a Periodically Poled Lithium Niobate (PPLN) crystal, and an optical fiber loop. The laser utilizes a femtosecond pulsed fiber laser matched with an amplifier system. The amplified laser first undergoes a frequency doubling process using the PPLN crystal. The frequency-doubled laser is used as a pump source to synchronously pump the PPLN crystal in the optical fiber loop to form degenerate optical parametric oscillation, and hundreds of oscillation pulses can exist simultaneously in the optical fiber loop. The electrical part includes an FPGA (Field Programmable Gate Array), AD/DA (digital-to-analog/analog-to-digital conversion), and a phase detection part. The laser output from the optical fiber loop and the fundamental frequency laser are measured by a phase detector, which can test the phase of the output light. The FPGA matched with high-speed AD/DA is utilized for optical pulse measurement and feedback control.

Unlike classical computers running on semiconductor integrated circuits, CIM uses laser pulses in optical fibers as quantum bits (qubits) for computation. In DOPO, the pump light is incident on a nonlinear optical crystal and splits into two beams. The two beams have the same polarization direction, a frequency half that of the pump light, are in a squeezed state, and can serve as a qubit. As the pump light power is gradually increased beyond the oscillation threshold, the generated light becomes a coherent state, and the phase of the light is divided into two states (phase 0 state and state π). At this time, the phase can be correspondingly set to ±1 of spins to solve optimization problems.

By solving through the photonic quantum computer, a set of solutions that minimize the target value *H* is obtained. This set of solutions is a set of binary variables 0 and 1, for example, in the form of [0, 1, 1, 1, 0, 0, 1, 0, ...]. The number of elements in this list is the number of vertices in the conflict graph. A 1 in the solution indicates that the corresponding vertex is included in the maximum weighted independent subset of the conflict graph, and 0 indicates that it is not in the maximum weighted independent subset of the conflict graph. Through the maximum weighted independent subset, the vertex information in the two molecular graphs can be obtained.

4. Calculate the similarity of the two molecules through the information of respective vertices of the conflict graph in the maximum weighted independent subset.

After the maximum weighted independent subset is found, the vertex information of the conflict graph included in the maximum weighted independent subset can be obtained, and the similarity of the two molecules can be calculated based on the vertex information. The calculation method is as follows: $S \left(G,G′\right) = δ max \left\{\frac{\left|{V}_{c}^{1}\right|}{\left|V1\right|}, \frac{\left|{V}_{c}^{2}\right|}{\left|V2\right|}\right\} + \left(1-δ\right) min \left\{\frac{\left|{V}_{c}^{1}\right|}{\left|V1\right|}, \frac{\left|{V}_{c}^{2}\right|}{\left|V2\right|}\right\} , δ ∈ \left[0, 1\right]$

Wherein, *G* and *G*' represent the two molecules requiring molecular similarity calculation, *S*(*G, G'*) represents the similarity between molecule *G* and molecule G', |*V*1| and |*V*2| respectively represent the number of atoms of molecule *G* and molecule G', $\left|{V}_{c}^{1}\right|$ and $\left|{V}_{c}^{2}\right|$ respectively represent the number of atoms belonging only to molecule G and only to molecule *G*' in the respective vertices of the conflict graph in the maximum weighted independent subset, and *δ* is an adjustable parameter.

For example, molecule *G* and molecule *G'* have 10 and 12 vertices (i.e., 10 and 12 atoms), respectively. In the obtained vertex set in the maximum weighted independent subset, there are 5 unique atoms belonging to molecule *G* and 4 unique atoms belonging to molecule *G*', then the similarity is calculated by substituting these values into the above formula: $S \left(G,G′\right) = δ max \left\{\frac{5}{10}, \frac{4}{12}\right\} + \left(1-δ\right) min \left\{\frac{5}{10}, \frac{4}{12}\right\} = 0.416$

Wherein, *δ* is an adjustable parameter, which is 0.5 in this example.

By designing a solving model suitable for a photonic quantum computer, the present disclosure solves the problem of molecular similarity calculation, overcomes the disadvantages of slow solving speed and high false positive rate of traditional solving methods, and effectively improves calculation speed and accuracy.

In order to verify the accuracy of the model for active molecule screening, the present disclosure utilizes this model combined with a photonic quantum computer for solving, and verified it on the dataset DUD_LIB_VS_1.0 library. This dataset is a calibrated dataset used to evaluate the performance of screening methods, containing multiple protein classes. Each target protein class contains a set of active molecules (activities) and a set of inactive molecules (decoys). Active molecules are molecules experimentally verified to bind to the target protein, while inactive molecules are molecules with similar structures but no relevant binding activity. In addition, it contains a query molecule, which is the ligand molecule included when resolving the target protein structure and has the optimal conformation for binding to the target protein. By calculating the similarity of the query molecule to the active molecule set and the inactive molecules, and screening according to a threshold, the calculation performance of the model is evaluated by finally calculating the screening accuracy and the screening effect of the head molecules.

The present disclosure selected the catechol-O-methyltransferase (COMT) protein class as an example to compare the effect solved based on a photonic quantum computer (i.e., the effect of the present disclosure) with the traditional molecular fingerprint-based similarity calculation method (MFP) and the graph-based similarity calculation method (GMS). COMT is a degrading enzyme of dopamine, one of the main regulatory pathways of dopamine in the brain, and has been confirmed as a drug biological target for treating various central and peripheral nervous system diseases, including Parkinson's disease, depression, schizophrenia, and other dopamine deficiency-related diseases. The present disclosure calculated the similarity of 8 active molecules and 144 inactive molecules of the COMT class, and the calculation results are shown in Table 1.

**Table 1 Molecular similarity calculation results of COMT class**

| | Molecular | Similarity | Class |
|---|---|---|---|
| 1 | comt_decoys87.mol | 0.46 | decoys |
| 2 | comt_decoys138.mo1 | 0.51 | decoys |
| 3 | comt_decoys273.mol | 0.54 | decoys |
| 4 | comt_decoys237.mol | 0.46 | decoys |
| 5 | comt_decoys319.mol | 0.53 | decoys |
| 6 | comt_decoys154.mol | 0.37 | decoys |
| 7 | comt_decoys106.mol | 0.43 | decoys |
| 8 | comt_decoys84.mol | 0.5 | decoys |
| 9 | comt_decoys320.mol | 0.44 | decoys |
| 10 | comt_decoys44.mol | 0.44 | decoys |
| 11 | comt_decoys140.mol | 0.4 | decoys |
| 12 | comt_decoys445.mol | 0.51 | decoys |
| 13 | comt_decoys323.mol | 0.53 | decoys |
| 14 | comt_decoys376.mol | 0.5 | decoys |
| 15 | comt_decoys280.mol | 0.59 | decoys |
| 16 | comt_decoys141.mol | 0.47 | decoys |
| 17 | comt_decoys80.mol | 0.52 | decoys |
| 18 | comt_decoys317.mol | 0.39 | decoys |
| 19 | comt_decoys406.mol | 0.4 | decoys |
| 20 | comt_decoys217.mol | 0.37 | decoys |
| 21 | comt_decoys79.mol | 0.42 | decoys |
| 22 | comt_decoys349.mol | 0.5 | decoys |
| 23 | comt_decoys333.mol | 0.44 | decoys |
| 24 | comt_decoys396.mol | 0.55 | decoys |
| 25 | comt_decoys435.mol | 0.49 | decoys |
| 26 | comt_decoys76.mol | 0.41 | decoys |
| 27 | comt_decoys291.mol | 0.58 | decoys |
| 28 | comt_decoys326.mol | 0.39 | decoys |
| 29 | comt_decoys229.mol | 0.5 | decoys |
| 30 | comt_decoys353.mol | 0.49 | decoys |
| 31 | comt_decoys18.mol | 0.4 | decoys |
| 32 | comt_decoys176.mol | 0.4 | decoys |
| 33 | comt_decoys432.mol | 0.41 | decoys |
| 34 | comt_decoys72.mol | 0.49 | decoys |
| 35 | comt_decoys160.mol | 0.37 | decoys |
| 36 | comt_decoys208.mol | 0.44 | decoys |
| 37 | comt_decoys73.mol | 0.53 | decoys |
| 38 | comt_decoys403.mol | 0.53 | decoys |
| 39 | comt_decoys233.mol | 0.49 | decoys |
| 40 | comt_decoys417.mol | 0.49 | decoys |
| 41 | comt_decoys465.mol | 0.53 | decoys |
| 42 | comt_decoys151.mol | 0.62 | decoys |
| 43 | comt_decoys115.mol | 0.62 | decoys |
| 44 | comt_decoys219.mol | 0.54 | decoys |
| 45 | comt_decoys234.mol | 0.52 | decoys |
| 46 | comt_decoys174.mol | 0.49 | decoys |
| 47 | comt_decoys177.mol | 0.59 | decoys |
| 48 | comt_decoys416.mol | 0.43 | decoys |
| 49 | comt_decoys287.mol | 0.44 | decoys |
| 50 | comt_decoys318.mol | 0.5 | decoys |
| 51 | comt_decoys25.mol | 0.57 | decoys |
| 52 | comt_decoys211.mol | 0.51 | decoys |
| 53 | comt_decoys70.mol | 0.41 | decoys |
| 54 | comt_decoys210.mol | 0.57 | decoys |
| 55 | comt_decoys17.mol | 0.47 | decoys |
| 56 | comt_decoys259.mol | 0.46 | decoys |
| 57 | comt_decoys450.mol | 0.6 | decoys |
| 58 | comt_decoys167.mol | 0.49 | decoys |
| 59 | comt_decoys255.mol | 0.56 | decoys |
| 60 | comt_decoys148.mol | 0.42 | decoys |
| 61 | comt_decoys153.mol | 0.57 | decoys |
| 62 | comt_decoys430.mol | 0.56 | decoys |
| 63 | comt_decoys5.mol | 0.45 | decoys |
| 64 | comt_decoys330.mol | 0.34 | decoys |
| 65 | comt_decoys440.mol | 0.36 | decoys |
| 66 | comt_decoys301.mol | 0.53 | decoys |
| 67 | comt_decoys60.mol | 0.64 | decoys |
| 68 | comt_decoys95.mol | 0.49 | decoys |
| 69 | comt_decoys328.mol | 0.67 | decoys |
| 70 | comt_decoys134.mol | 0.55 | decoys |
| 71 | comt_decoys412.mol | 0.52 | decoys |
| 72 | comt_decoys172.mol | 0.36 | decoys |
| 73 | comt_decoys251.mol | 0.51 | decoys |
| 74 | comt_decoys257.mol | 0.48 | decoys |
| 75 | comt_decoys16.mol | 0.42 | decoys |
| 76 | comt_decoys411.mol | 0.52 | decoys |
| 77 | comt_decoys155.mol | 0.44 | decoys |
| 78 | comt_decoys385.mol | 0.49 | decoys |
| 79 | comt_decoys175.mol | 0.48 | decoys |
| 80 | comt_decoys163.mol | 0.47 | decoys |
| 81 | comt_decoys53.mol | 0.53 | decoys |
| 82 | comt_decoys58.mol | 0.57 | decoys |
| 83 | comt_decoys220.mol | 0.46 | decoys |
| 84 | comt_decoys39.mol | 0.58 | decoys |
| 85 | comt_decoys437.mol | 0.64 | decoys |
| 86 | comt_decoys467.mol | 0.41 | decoys |
| 87 | comt_decoys126.mol | 0.44 | decoys |
| 88 | comt_decoys426.mol | 0.45 | decoys |
| 89 | comt_decoys45.mol | 0.5 | decoys |
| 90 | comt_decoys400.mol | 0.56 | decoys |
| 91 | comt_decoys183.mol | 0.56 | decoys |
| 92 | comt_decoys418.mol | 0.41 | decoys |
| 93 | comt_decoys11.mol | 0.42 | decoys |
| 94 | comt_decoys266.mol | 0.44 | decoys |
| 95 | comt_decoys122.mol | 0.42 | decoys |
| 96 | comt_decoys123.mol | 0.45 | decoys |
| 97 | comt_decoys43.mol | 0.47 | decoys |
| 98 | comt_decoys157.mol | 0.35 | decoys |
| 99 | comt_decoys30.mol | 0.47 | decoys |
| 100 | comt_decoys156.mol | 0.35 | decoys |
| 101 | comt_decoys158.mol | 0.35 | decoys |
| 102 | comt_decoys382.mol | 0.41 | decoys |
| 103 | comt_decoys99.mol | 0.53 | decoys |
| 104 | comt_decoys165.mol | 0.44 | decoys |
| 105 | comt_decoys109.mol | 0.39 | decoys |
| 106 | comt_decoys54.mol | 0.38 | decoys |
| 107 | comt_decoys97.mol | 0.44 | decoys |
| 108 | comt_decoys125.mol | 0.46 | decoys |
| 109 | comt_decoys49.mol | 0.46 | decoys |
| 110 | comt_decoys36.mol | 0.42 | decoys |
| 111 | comt_decoys254.mol | 0.47 | decoys |
| 112 | comt_decoys454.mol | 0.53 | decoys |
| 113 | comt_decoys199.mol | 0.47 | decoys |
| 114 | comt_decoys162.mol | 0.58 | decoys |
| 115 | comt_decoys351.mol | 0.41 | decoys |
| 116 | comt_decoys455.mol | 0.52 | decoys |
| 117 | comt_decoys129.mol | 0.49 | decoys |
| 118 | comt_decoys394.mol | 0.51 | decoys |
| 119 | comt_decoys47.mol | 0.49 | decoys |
| 120 | comt_decoys325.mol | 0.65 | decoys |
| 121 | comt_decoys137.mol | 0.45 | decoys |
| 122 | comt_decoys136.mol | 0.46 | decoys |
| 123 | comt_decoys252.mol | 0.5 | decoys |
| 124 | comt_decoys242.mol | 0.47 | decoys |
| 125 | comt_decoys262.mol | 0.53 | decoys |
| 126 | comt_decoys375.mol | 0.47 | decoys |
| 127 | comt_decoys267.mol | 0.47 | decoys |
| 128 | comt_decoys132.mol | 0.41 | decoys |
| 129 | comt_decoys31.mol | 0.46 | decoys |
| 130 | comt_decoys373.mol | 0.56 | decoys |
| 131 | comt_decoys216.mol | 0.39 | decoys |
| 132 | comt_decoys192.mol | 0.51 | decoys |
| 133 | comt_decoys379.mol | 0.65 | decoys |
| 134 | comt_decoys352.mol | 0.46 | decoys |
| 135 | comt_decoys345.mol | 0.5 | decoys |
| 136 | comt_decoys41.mol | 0.5 | decoys |
| 137 | comt_decoys187.mol | 0.58 | decoys |
| 138 | comt_decoys100.mol | 0.46 | decoys |
| 139 | comt_decoys264.mol | 0.38 | decoys |
| 140 | comt_decoys245.mol | 0.49 | decoys |
| 141 | comt_decoys409.mol | 0.55 | decoys |
| 142 | comt_decoys408.mol | 0.5 | decoys |
| 143 | comt_decoys423.mol | 0.41 | decoys |
| 144 | comt_decoys249.mol | 0.51 | decoys |
| 145 | comt_activities1.mol | 0.68 | activities |
| 146 | comt_activities2.mol | 0.62 | activities |
| 147 | comt_activities3.mol | 0.72 | activities |
| 148 | comt_activities4.mol | 0.61 | activities |
| 149 | comt_activities5.mol | 0.67 | activities |
| 150 | comt_activities6.mol | 0.69 | activities |
| 151 | comt_activities7.mol | 0.68 | activities |
| 152 | comt_activities8.mol | 0.6 | activities |

In the biomedical field, the quality of a screening model is often characterized by the Receiver Operating Characteristic curve (ROC curve). This curve sets multiple different critical values for continuous variables, calculates the corresponding sensitivity and specificity at each critical value, and then plots the curve with sensitivity as the ordinate and 1-specificity as the abscissa. The area under the ROC curve (AUC) can be used as a quantitative indicator to evaluate the classification performance of the model. The ROC curve of the molecular similarity model solved based on the photonic quantum computer for the COMT class is shown in Fig. 2, where the AUC reached 98%. Therefore, for the screening of the entire dataset, the model has achieved a very ideal effect.

In the pharmaceutical field, virtual screening of large compound libraries can only perform biological activity testing on a small fraction of the compounds. Therefore, in addition to the classification effect on the entire dataset, the early enrichment capability of the model's virtual screening may be of greater concern. ROCEnrichment0.5% (ROCE0.5%) and ROCEnrichment1% (ROCE1%) respectively represent the ratio of the true positive rate to the false positive rate when the false positive rate is 0.5% and 1%. A larger value indicates better early enrichment capability. Through calculation, the ROCE0.5% and ROCE1% of the COMT class solved based on the photonic quantum computer are 125 and 62.5, respectively, which are superior to the ROCE0.5% and ROCE1% of the traditional MFP method and the graph-based similarity calculation method GMS (Table 2).

**Table 2 Comparison of effects among QMS, GMS, and MFP methods**

| Method | AUC | ROCE0.5% | ROCE1% |
|---|---|---|---|
| **QMS** | **0.98** | **125.00** | **62.50** |
| GMS | - | 70.91 | 51.06 |
| MFP | - | 0.00 | 8.51 |

Finally, the present disclosure further relates to a molecular similarity calculation apparatus based on a photonic quantum computer, including: one or more processors; a memory, configured to store one or more programs; wherein when the one or more programs are executed by the one or more processors, the one or more processors are caused to implement the molecular similarity calculation method based on a photonic quantum computer as described above.

Finally, it should be noted that the above embodiments are merely used to illustrate the technical solutions of the present disclosure, rather than limiting the protection scope of the present disclosure. Those skilled in the art, based on the idea of the present disclosure, can modify or equivalently replace the technical solutions of the present disclosure without departing from the essence and scope of the technical solutions of the present disclosure.

## Claims

1. A molecular similarity calculation method based on photonic quantum computer, comprising:
converting two molecules requiring molecular similarity calculation into molecular graphs respectively based on molecular structures, wherein vertices of the molecular graph represent respective atoms in the molecule, and edges of the molecular graph represent chemical bond connections between respective atoms in the molecule;
constructing a conflict graph utilizing the two molecular graphs, wherein vertices of the conflict graph are derived from pairwise matching mappings between vertices in the two molecular graphs, and edges of the conflict graph are derived from discrimination results between respective vertices in the conflict graph;
solving a maximum weighted independent subset of the conflict graph by the photonic quantum computer and obtaining information of respective vertices of the conflict graph in the maximum weighted independent subset;
calculating the similarity of the two molecules through the information of respective vertices of the conflict graph in the maximum weighted independent subset.

2. The molecular similarity calculation method based on photonic quantum computer according to claim 1, wherein the vertices of the molecular graph contain characteristic information of atoms and the edges of the molecular graph contain chemical bond connection information between atoms, wherein the characteristic information of the atoms includes atom name, number of explicit hydrogens, number of implicit hydrogens, number of adjacent atoms, explicit valence, formal charge, and whether it is an atom in a ring, and the chemical bond connection information between atoms includes bond type, bond aromaticity, whether it is a bond in a ring, and bond stereo information.

3. The molecular similarity calculation method based on photonic quantum computer according to claim 1, wherein when constructing the conflict graph utilizing the two molecular graphs, matched atom pairs are obtained by comparing the similarity between respective atoms of the original molecules corresponding to the two molecular graphs, and the matched atom pairs are used as the vertices of the conflict graph; and whether mappings of two vertices in the conflict graph conflict is obtained by comparing whether a difference between distances of two atoms in the original molecules corresponding to the vertices in the two molecular graphs contained in the two vertices of the conflict graph is greater than a threshold, thereby determining whether to add an edge between the two vertices.

4. The molecular similarity calculation method based on photonic quantum computer according to claim 3, wherein obtaining matched atom pairs by comparing the similarity between respective atoms of the original molecules corresponding to the two molecular graphs, and using the matched atom pairs as the vertices of the conflict graph specifically comprises:
calculating the similarity between respective atoms of the original molecules corresponding to the two molecular graphs, wherein the similarity between two atoms is calculated as follows: $similarity between two atoms = Average \left({\sum }_{n = 1}^{N} \frac{min \left({Gf}_{n},G′{f}_{n}\right)}{max \left({Gf}_{n},G′{f}_{n}\right)}\right)$ wherein, *N* represents the total number of the atom's characteristic information, *n* represents the ordinal number of the atom's characteristic information, *Gfₙ* and *G'fₙ* respectively represent a value of an n-th characteristic information of the atom in the original molecule corresponding to a first molecular graph and a value of an *n*-th characteristic information of the atom in the original molecule corresponding to a second molecular graph, min(*Gfₙ*, *G'fₙ*) represents a smaller value between the value of the *n*-th characteristic information of the atom in the original molecule corresponding to the first molecular graph and the value of the n-th characteristic information of the atom in the original molecule corresponding to the second molecular graph, and *max*(*Gfₙ, G'fₙ*) represents a larger value therebetween;
comparing the similarity between the two atoms with a threshold S, and when it is greater than the threshold S, determining that a mapping exists between the atom in the original molecule corresponding to the first molecular graph and the atom in the original molecule corresponding to the second molecular graph, whereby these two atoms are a matched atom pair, and taking the matched atom pair as a vertex of the conflict graph;
performing pairwise atom similarity calculations on all atoms in the original molecule corresponding to the first molecular graph and all atoms in the original molecule corresponding to the second molecular graph to find all matched atom pairs, and obtaining a vertex set with vertex weights $\left\{\left(vi,v′j\right)\left|{\sum }_{n = 1}^{N} {Gf}_{n} + G ′ {f}_{n}\right.\right\}$ of the conflict graph, wherein *vᵢ* represents the i-th atom in the original molecule corresponding to the first molecular graph, *v'ⱼ* represents the j-th atom in the original molecule corresponding to the second molecular graph, and ${\sum }_{n = 1}^{N} {Gf}_{n} + G ′ {f}_{n}$ represents the vertex weight of the vertex in the conflict graph.

5. The molecular similarity calculation method based on photonic quantum computer according to claim 4, wherein the threshold *S* is 0.75.

6. The molecular similarity calculation method based on photonic quantum computer according to claim 5, wherein obtaining whether mappings of two vertices in the conflict graph conflict by comparing whether the difference between the distances of two atoms in the original molecules corresponding to the vertices in the two molecular graphs contained in the two vertices of the conflict graph is greater than a threshold, thereby determining whether to add an edge between the two vertices, specifically comprises:
comparing whether the difference between the distances of the two atoms in the original molecules corresponding to the vertices in the two molecular graphs contained in the two vertices of the conflict graph is greater than a threshold *dₜ,* and if it is greater than the threshold *dₜ,* adding an edge between the two vertices, wherein a specific mathematical expression for the determination is as follows: ${E}_{c} = \left\{\left.\left({v}_{i},v{′}_{j}\right), \left({v}_{k},v{′}_{l}\right)\right|\left|d\left({v}_{i}, {v}_{k}\right)-d\left(v{′}_{j},v{′}_{l}\right)\right|>{d}_{t}\right\}$ wherein, *E_{c}* represents an edge set between the vertices of the conflict graph, (*vᵢ, v'ⱼ*) and (*vₖ, v'ₗ*) represent two vertices in the conflict graph, and *d*(*vᵢ, vₖ*) and *d*(*v'ⱼ, v'ₗ*) respectively represent a geometric distance between two atoms in the original molecule corresponding to the vertices *vᵢ* and *vₖ* in the first molecular graph and a geometric distance between two atoms in the original molecule corresponding to the vertices *v'ⱼ* and *v'ₗ* in the second molecular graph.

7. The molecular similarity calculation method based on photonic quantum computer according to claim 6, wherein the threshold *dₜ* is 2, and an edge weight is assigned to an edge of the conflict graph, wherein a value of the edge weight is a smaller value of the vertex weights of the two connected vertices plus 1.

8. The molecular similarity calculation method based on photonic quantum computer according to claim 7, wherein when solving the maximum weighted independent subset of the conflict graph, a QUBO model as follows is constructed: $H = min \left(-{\sum }_{p ∈ Vc} {w}_{p} {x}_{p} + {\sum }_{\left(p, q\right) ∈ {E}_{c}} {w}_{pq} {x}_{p} {x}_{q}\right)$ wherein, *H* represents the Hamiltonian, which is an optimization target value; *p* and q respectively represent a *p*-th vertex and a *q*-th vertex in the conflict graph; *xₚ* and *x_{q}* are two binary variables, if the *p-*th vertex and the *q*-th vertex of the conflict graph are respectively included in the maximum weighted independent subset, *xₚ* and *x_{q}* are equal to 1, otherwise *xₚ* and *x_{q}* are equal to 0; *wₚ* is the vertex weight of the *p*-th vertex in the conflict graph; *w_{pq}* represents the edge weight of the edge connecting the *p*-th vertex and the *q*-th vertex in the conflict graph; and *V_{c}* and *E_{c}* respectively represent the vertex set and the edge set in the conflict graph.

9. The molecular similarity calculation method based on photonic quantum computer according to claim 8, wherein a specific calculation method for calculating the similarity of the two molecules through the information of respective vertices of the conflict graph in the maximum weighted independent subset is as follows: $S \left(G,G′\right) = δ max \left\{\frac{\left|{V}_{c}^{1}\right|}{\left|V1\right|}, \frac{\left|{V}_{c}^{2}\right|}{\left|V2\right|}\right\} + \left(1-δ\right) min \left\{\frac{\left|{V}_{c}^{1}\right|}{\left|V1\right|}, \frac{\left|{V}_{c}^{2}\right|}{\left|V2\right|}\right\} , δ ∈ \left[0, 1\right]$ wherein, G and G' represent the two molecules requiring molecular similarity calculation, *S*(*G, G')* represents the similarity between molecule *G* and molecule *G'*, |*V*1| and |*V*2| respectively represent the number of atoms of molecule *G* and molecule *G*', $\left|{V}_{c}^{1}\right|$ and $\left|{V}_{c}^{2}\right|$ respectively represent the number of atoms belonging only to molecule *G* and only to molecule *G*' in the respective vertices of the conflict graph in the maximum weighted independent subset, and *δ* is an adjustable parameter.

10. A molecular similarity calculation apparatus based on photonic quantum computer, comprising:
one or more processors;
a memory, configured to store one or more programs;
wherein when the one or more programs are executed by the one or more processors, the one or more processors are caused to implement the molecular similarity calculation method based on photonic quantum computer according to any one of claims 1-9.
